(19) Europäisches Patentamt
European Patent Office
Office européen des brevets

(11) Numéro de publication : **0 165 225**
**B1**

(12) # FASCICULE DE BREVET EUROPÉEN

(45) Date de publication du fascicule du brevet : **23.03.88**

(51) Int. Cl.[4] : **C 07 D471/04**, C 07 D213/89 // (C07D471/04, 221:00, 209:00)

(21) Numéro de dépôt : **85870072.7**

(22) Date de dépôt : **23.05.85**

(54) **Procédé de préparation de l'aza-5 indole et intermédiaires.**

(30) Priorité : **25.05.84 FR 8408275**

(43) Date de publication de la demande : **18.12.85 Bulletin 85/51**

(45) Mention de la délivrance du brevet : **23.03.88 Bulletin 88/12**

(84) Etats contractants désignés : **AT BE CH DE FR GB IT LI LU NL SE**

(56) Documents cités :
**JOURNAL OF MEDICINAL CHEMISTRY, vol. 15, no. 11, novembre 1972, pages 1168-1171; Washington D.C., US;M.J. FISHER et al.: "Azaindole anthelmintic agents"**
**CHEMICAL ABSTRACTS, vol. 87, no. 25, 19 décembre 1977, page 714, résumé no. 201382w, Columbus, Ohio, US; A.A. PROKOPOV et al.: "Synthesis of 6-azaindole"**
**CHEM. BER. 101 (1968, S. 4048-4056**

(73) Titulaire : **SANOFI, société anonyme**
**40, Avenue George V**
**F-75008 Paris (FR)**

(72) Inventeur : **Dormoy, Jean-Robert**
**Avenue du Thor, 9**
**F-04200 Sisteron (FR)**
Inventeur : **Heymes, Alain**
**Rue Frédéric Mistral "Les Plantiers"**
**F-04200 Sisteron (FR)**

(74) Mandataire : **Moncheny, Michel et al**
**c/o Cabinet Lavoix 2 Place d'Estienne d'Orves**
**F-75441 Paris Cedex 09 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 165 225 B1

**Description**

La présente invention se rapporte, d'une manière générale, à un nouveau procédé de préparation d'un dérivé de pyridine ainsi qu'au dérivé obtenu par ce procédé.

L'invention concerne notamment un nouveau procédé pour la préparation de la 1H-pyrrolo-[3,2-c] pyridine ou aza-5 indole de formule :

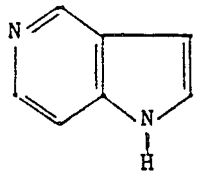

(I)

Ce composé est particulièrement utile comme intermédiaire de synthèse pour la préparation de composés biologiquement actifs par exemple pour la préparation des dérivés antiinflammatoires amino-4 aryle aza-5 indole décrits dans Chimie Thérapeutique 5, 559-566 (1973).

Les méthodes décrites dans la littérature chimique pour la préparation de l'aza-5 indole sont essentiellement du domaine du laboratoire et difficilement extrapolables sur le plan industriel.

Par exemple, on a décrit dans Ann. Chem., 612, 153-7 (1958) une méthode de synthèse de ce composé faisant appel à une réduction photochimique de cycle de naphtyridine. Cette étape de réduction photochimique est difficilement transportable à des quantités importantes de produit. Par ailleurs, le rendement global en aza-5 indole n'est que de 11 %.

On a cité en outre dans J. Org. Chem. 30, 2531-2533 (1965) une autre méthode de préparation de l'aza-5 indole à partir de méthyl-3 amino-4 pyridine.

La synthèse décrite est la plus efficace de celles rapportées dans la littérature. Cependant, le rendement global de 36 % et en particulier celui de la dernière étape, n'a pu être reproduit ni par d'autres expérimentateurs ni dans le cadre de la présente invention.

D'autres procédés ont été proposés notamment dans Tetrahedron Letters, 24, 1909-1912 (1969) mais ils paraissent peu intéressants si l'on considère l'accessibilité des produits de départ.

Par conséquent, la recherche d'un procédé industriel pour l'obtention de l'aza-5 indole capable de remédier aux inconvénients et désavantages des méthodes antérieures reste d'un intérêt primordial.

Yakontov et coll. ont décrit dans Khim. Geterotsikl. Soedin, 10, 1425 (1977) (CA 88, 37665p) la préparation de l'aza-4 indole et dans Khim. Geterotsikl. Soedin, 8, 1135-1136 (1977) (CA 87, 201382 w) la préparation de l'aza-6 indole faisant intervenir, dans une première étape, la formation d'une énamine.

Suivant cette méthode, les aza-4 et aza-6 indoles sont obtenus à partir respectivement de méthyl-2 nitro-3 pyridine et de méthyl-4 nitro-3 pyridine : ces dérivés de pyridine sont condensés avec un acétal de formamide pour former l'O-nitro-β-diméthylaminovinyl pyridine correspondante qui est ensuite réduite sur catalyseur palladié en aza indole correspondant.

Les rendements rapportés, pour chacun de ces aza indoles, sont de l'ordre de 80 à 100 % calculés à partir du dérivé de méthyl pyridine.

Etant donné la similitude de structure chimique entre l'aza-5 indole et les aza-4 et aza-6 indoles, on a tenté, dans le cadre de la présente invention, de transposer, à la méthyl-3 nitro-4 pyridine, le procédé décrit précédemment en vue de l'obtention finale d'aza-5 indole.

Cependant, les rendements globaux obtenus se sont révélés relativement faibles, de l'ordre de 50 % calculés à partir du dérivé de méthyl pyridine.

Il apparaît, par conséquent, que l'extrapolation pure et simple de la technique antérieure n'est pas envisageable pour la préparation de l'aza-5 indole selon un procédé exploitable industriellement.

Par conséquent, il reste essentiel de trouver un procédé de préparation de l'aza-5 indole présentant les qualités suivantes :

— simplicité de mise en œuvre
— rendements plus élevés
— prix de revient aussi faible que possible.

On a maintenant découvert, suivant la présente invention, qu'il est possible d'obtenir l'aza-5 indole selon un tel procédé industriel en mettant en œuvre la méthyl-3 nitro-4-pyridine-1-oxyde en tant que dérivé de méthyl pyridine de départ pour la formation d'une énamine et sa transformation ultérieure en aza indole désiré.

Cette constatation est, par ailleurs, surprenante à la lumière de Chem. Ber. 101, 4048-4056 (1968) qui suggère, de manière évidente, l'utilisation d'une méthyl pyridine non oxydée pour la formation d'une énamine plutôt que du dérivé méthyl pyridine N-oxyde correspondant.

Par exemple, on y a rapporté l'obtention de (diméthylamino-2 vinyl)-4 pyridine avec un rendement de 84 % à partir de méthyl-4 pyridine et d'un acétal de formamide alors que la (diméthylamino-2 vinyl)-4 pyridine-N-oxyde n'a pu être fournie qu'avec un rendement de 18 % à partir de méthyl-4 pyridine N-oxyde.

Ainsi, un premier objet de l'invention concerne un procédé de préparation de l'aza-5 indole selon lequel

on condense à une température comprise entre 0 et 200 °C généralement entre 100 et 150 °C, la méthyl-3 nitro-4 pyridine-1-oxyde de formule :

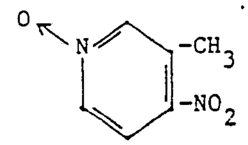

(II)

avec un composé de formule générale :

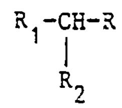

(III)

dans laquelle R représente un groupement diméthylamino, diéthylamino, dipropylamino, dibutylamino, morpholino, pipéridino ou pyrrolidino et $R_1$ et $R_2$, qui sont identiques ou différents, représentent chacun un groupement méthoxy, éthoxy ou propoxy ou un groupement R tel que défini précédemment, pour obtenir une énamine de formule générale :

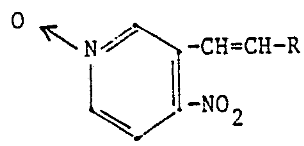

(IV)

dans laquelle R a la même valeur que précédemment, puis on effectue dans un solvant une réduction cyclisation par l'hydrogène en présence d'un catalyseur tel que, par exemple, le nickel de Raney ou le charbon palladié, pour obtenir l'aza-5 indole désiré.

Un autre objet de l'invention se rapporte aux énamines de formule IV en tant que produits industriels pour la préparation de l'aza-5 indole selon le procédé ci-dessus.

La formation des énamines en question sera effectuée en l'absence de solvant ou préférentiellement dans un solvant approprié tel que le N,N-diméthylformamide ou un alcool inférieur tel que le méthanol ou l'éthanol.

La réduction cyclisation sera entreprise dans un alcool inférieur par exemple l'éthanol, dans un glycol, dans l'acide acétique ou dans le N,N-diméthylformamide à une température comprise entre 0 et 120 °C généralement à une température voisine de 60 °C.

Suivant le procédé de l'invention ainsi décrit, l'aza-5 indole peut être obtenu avec des rendements de l'ordre de 80 % calculés à partir du composé de formule II. Globalement, le procédé de l'invention se révèle plus simple et plus rentable que celui suggéré par Yakontov et coll.

En fait, l'application du procédé de Yakontov et coll. à la synthèse de l'aza-5 indole nécessiterait la préparation de la méthyl-3 nitro-4 pyridine. Ce composé est généralement obtenu à partir de la méthyl-3 nitro-4 pyridine-1-oxyde, qui est le composé de formule II ci-dessus, par réduction au moyen, par exemple, de trichlorure de phosphore.

Le procédé de l'invention évite cette étape de réduction intermédiaire, la réduction du groupement N-oxyde du composé de formule II s'effectuant lors de la réduction cyclisation finale.

On a comparé, dans le cadre de la présente invention, deux procédés partant chacun de méthyl-3 nitro-4 pyridine-1-oxyde, l'un suivant les conditions opératoires selon l'invention, l'autre suivant une mise en œuvre analogue à celle de Yakontov et coll. c'est-à-dire en incluant l'étape transitoire de réduction de ce composé N-oxyde en méthyl-3 nitro-4 pyridine.

Dans le premier cas, le rendement en aza-5 indole s'est révélé être de l'ordre de 80 %, dans l'autre le rendement n'était que de 44 %.

Le procédé de l'invention s'avère donc, dans l'ensemble, plus économique que celui suggéré par l'art antérieur.

De plus, suivant une mise en œuvre particulière du procédé de l'invention, en utilisant comme source de composé de formule III un adduit résultant de la réaction de la morpholine sur un orthoformiate de méthyle ou d'éthyle, on réalise l'étape de formation de l'énamine de formule IV au sein du milieu même de formation de l'adduit.

Dans ce but, on utilise une stoechiométrie appropriée de réactifs, généralement un rapport molaire allant de 3 à 9/1,5 à 4,5, de préférence un rapport molaire de 6/3, en morpholine/orthoformiate d'alkyle par mole de méthyl-3 nitro-4 pyridine-1-oxyde, pour obtenir des rendements globaux d'au moins 70 % en aza-5 indole.

L'utilisation d'un tel adduit morpholine/orthoformiate d'alkyle présente en outre certains avantages

non négligeables qui rendent cette mise en œuvre préférée.

En effet, les autres composés de formule III préalablement isolés tels que les acétals diméthylique ou diéthylique du N,N-diméthylformamide nécessitent une synthèse en deux étapes à savoir la chloruration du N,N-diméthylformamide puis l'éthérification par le méthylate ou l'éthylate de sodium, suivie d'une distillation finale.

L'obtention d'énamine à partir de ces acétals s'effectue donc suivant un processus en trois étapes.

Par contre, la mise en œuvre préférée selon l'invention conduit à l'obtention de l'énamine en deux étapes chimiques dans le même milieu réactionnel sans isolation ni purification de l'adduit intermédiaire morpholine/orthoformiate d'alkyle résultant de la première étape chimique. Cette technique n'a pas été décrite par Yakontov et coll., dans les références précédemment citées, lesquels utilisent des acétals de formamide préalablement isolés. Au surplus, l'adduit en question peut être préféré au trismorpholinométhane, autre composé de formule III, dont la conservation peut être rendue difficile en raison de son hygroscopicité.

Ainsi, la mise en œuvre préférée, selon l'invention, permet d'obtenir des rendements en aza-5 indole comparables à ceux obtenus à partir de composés de formule III préalablement isolés mais selon une technique simplifiée et encore plus économique.

Les composés de formule III ci-dessus, par exemple, l'acétal diméthylique du N,N-diméthylformamide, l'acétal diéthylique du N,N-diéthylformamide et le trismorpholinométhane, sont des composés connus ou pouvant être préparés par des méthodes connues telles que celles décrites dans Angew. Chem. 72, 836-845 (1960), Helv. Chim. Acta, 44 (5), 1203-1211 (1961) ou dans la demande de brevet européen No. 001633.

Quant à la méthyl-3 nitro-4 pyridine-1-oxyde de formule II, on peut l'obtenir après nitration de la méthyl-3 pyridine-1-oxyde elle même préparée par oxydation de la méthyl-3 pyridine.

Les Exemples, non limitatifs suivants, illustrent l'invention :

## Exemple 1

Préparation de la 1H-pyrrolo-[3,2-c]-pyridine ou aza-5 indole.

a) (β-Diméthylaminovinyl)-3 nitro-4 pyridine-1-oxyde

On met en solution dans 10 ml de N,N-diméthylformamide, 77 g (0,5 mole) de méthyl-3 nitro-4 pyridine-1-oxyde et 80 ml (0,6 mole) d'acétal diméthylique de N,N-diméthylformamide.

On place le mélange sous courant d'azote sec et, sous agitation, on le porte progressivement à 120 °C. On maintient le chauffage pendant 2 à 3 heures à 120 °C période durant laquelle le méthanol libéré par la réaction distille. On élimine alors le méthanol, l'acétal diméthylique de N,N-diméthylformamide et le N,N-diméthylformamide sous pression réduite (60 °C, 4 mm Hg ou 5,32 m bar).

On reprend, dans l'éthanol, le solide brun-rouge résiduel et on précipite la fraction redissoute par addition d'éther éthylique. On essore les cristaux obtenus, lave à l'éther éthylique et sèche sous vide.

On obtient ainsi 99 g de (β-diméthylaminovinyl)-3 nitro-4 pyridine-1-oxyde sous forme de cristaux bruns-violets.

Rendement : 95 %
P.F. : 213-214 °C.
Analyse centésimale

| | | | |
|---|---|---|---|
| Calculé : | C 51,67 | H 5,26 | N 20,09 % |
| Trouvé : | C 51,67 | H 5,11 | N 20,39 % |

Spectre R.M.N. ($CDCl_3$/TMS)
3,0 (s, 6H) ; 5,9 (d, 1H, J = 13Hz) ; 7,4 (d, 1H, J = 13Hz) ; 7,5-8,0 (m,2H)
Le couplage de 13Hz indique une stéréochimie trans de la liaison de l'énamine.

b) 1H-pyrrolo-[3,2-c]-pyridine

Dans un appareil de PARR, fonctionnant à pression atmosphérique, on place 2,1 g (0,01 mole) de (β-diméthylaminovinyl)-3 nitro-4 pyridine-1-oxyde, 3,15 g de nickel de Raney (à 50 % dans l'eau) et 25 ml d'éthanol. On agite le mélange vigoureusement à température ambiante pendant 14h puis à 60 °C jusqu'à cessation de l'absorption d'hydrogène.

On essore le catalyseur sur Celite ® (produit commercial à base de terre d'infusoires) et on lave plusieurs fois à l'éthanol. On décolore la solution éthanolique sur charbon actif, on concentre sous vide et on filtre rapidement sur silice. Après évaporation du solvant, on obtient 0,98 g de 1H-pyrrolo-[3,2-c]-pyridine sous forme de cristaux d'un blanc jaunâtre.

Rendement : 84 %
P.F. : 110-122 °C.

Exemple 2

Préparation de l'aza-5 indole

a) (β-N-Morpholinovinyl)-3 nitro-4 pyridine-1-oxyde

On met en solution dans 20 ml de N,N-diméthylformamide, 7,7 g (0,05 mole) de méthyl-3 nitro-4 pyridine-1-oxyde et 16,3 g (0,06 mole) de trismorpholinométhane. On place le mélange sous courant d'azote sec et on chauffe à 100 °C. On maintient cette température pendant 2h.

Un produit rouge sombre cristallise par refroidissement de la solution de N,N-diméthylformamide. On essore ce produit, lave au méthanol et sèche sous vide.

On obtient ainsi 11,55 g de (β-N-morpholinovinyl)-3 nitro-4 pyridine-1-oxyde.

Rendement : 92 %

P.F. : 230 °C.

b) Aza-5 indole

Dans un appareil de PARR, fonctionnant à pression atmosphérique, on place 2,5 g (0,010 mole) de [β-N-morpholinovinyl]-3 nitro-4 pyridine-1-oxyde, 3,15 g de nickel de Raney (à 50% dans l'eau) et 25 ml d'éthanol. On agite vigoureusement le mélange à température ambiante pendant 15h puis à 60 °C jusqu'à cessation de l'absorption de l'hydrogène.

On essore le catalyseur sur Celite et on lave plusieurs fois à l'éthanol. On décolore la solution éthanolique sur charbon actif et on concentre sous vide. Après filtration rapide du résidu sur silice, on obtient 1g d'aza-5 indole sous forme de cristaux jaunes.

Rendement : 85%

P.F. : 109-110°C

Exemple 3

Préparation de l'aza-5 indole

a) (β-N-Morpholinovinyl)-3 nitro-4 pyridine-1-oxyde.

On met en solution, dans 5 ml de N,N-diméthylformamide, 8,9 g (0,06 mole) d'orthoformiate d'éthyle, 10,45g de morpholine (0,12 mole) et 0,36g (0,006 mole) d'acide acétique glacial.

On place le mélange sous atmosphère d'azote sec et on chauffe progressivement jusqu'à 140°C durant 45 min. jusqu'à cessation de la distillation de l'éthanol libéré par la création.

Dans le mélange préalablement refroidi, on introduit 3,08g (0,02 mole) de méthyl-3 nitro-4 pyridine-1-oxyde et on porte la température à 140°C. On maintient le milieu réactionnel à cette température pendant 90 min. puis on refroidit et évapore partiellement l'excès de réactif sous pression réduite. On essore les cristaux de couleur rouge sombre obtenus, lave au méthanol et sèche sous vide.

On obtient ainsi 4,2 g de (β-N-morpholinovinyl)-3 nitro-4 pyridine-1-oxyde.

Rendement : 84%

P.F. : 228°C.

b) Aza-5 indole

En suivant la méthode décrite dans l'Exemple 2b on obtient l'aza-5 indole avec un rendement de 85%.

**Revendications**

1. Procédé de préparation de la 1H-pyrrolo-[3,2-c] pyridine ou aza-5 indole de formule :

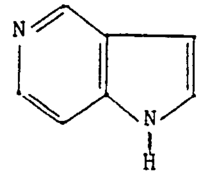

(I)

caractérisé en ce que l'on condense dans un solvant ou en l'absence de solvant la méthyl-3 nitro-4 pyridine-1-oxyde de formule :

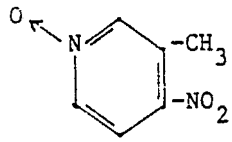

(II)

avec un composé de formule générale :

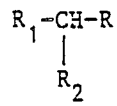

(III)

dans laquelle R représente un groupement diméthylamino, diéthylamino, dipropylamino, dibutylamino, morpholino, pipéridino ou pyrrolidino et $R_1$ et $R_2$, qui sont identiques ou différents, représentent chacun un groupement méthoxy, éthoxy ou propoxy ou un groupement R tel que défini précédemment, pour obtenir une énamine de formule générale :

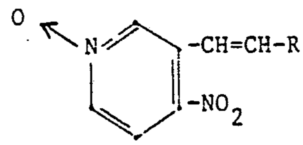

(IV)

dans laquelle R a la même valeur que précédemment puis on effectue dans un solvant une réduction cyclisation par l'hydrogène en présence d'un catalyseur pour obtenir l'aza-5 indole désiré.

2. Procédé selon la Revendication 1 caractérisé en ce que la condensation à lieu à une température comprise entre 0 et 200°C et la réduction cyclisation à une température comprise entre 0 et 120°C.

3. Procédé selon la Revendication 2 caractérisé en ce que la condensation a lieu à une température comprise entre 100 et 150°C et la réduction cyclisation à une température voisine de 60°C.

4. Procédé selon la Revendication 1 caractérisé en ce que la condensation a lieu dans le N,N-diméthylformamide et la réduction cyclisation dans l'éthanol comme solvants.

5. Procédé selon la Revendication 1 caractérisé en ce que le catalyseur est le nickel de Raney ou le charbon palladié.

6. Procédé selon l'une quelconque des Revendications 1 à 5 caractérisé en ce que le composé de formule III est l'acétal diméthylique du N,N-diméthylformamide, l'acétal diéthylique du N,N-diméthylformamide ou le trismorpholinométhane.

7. Procédé selon la Revendication 1 caractérisé en ce que l'on utilise comme source de composé de formule III un adduit, résultant de la réaction de la morpholine sur un orthoformiate de méthyle ou d'éthyle, que l'on condense avec la méthyl-3 nitro-4 pyridine-1-oxyde au sein du milieu de formation de l'adduit pour obtenir l'énamine de formule IV.

8. Procédé selon la Revendication 7 caractérisé en ce que l'on utilise un adduit résultant de la réaction de 3 à 9 moles de morpholine sur 1,5 à 4,5 moles d'orthoformiate de méthyle ou d'éthyle, par mole de méthyl-3 nitro-4 pyridine-1-oxyde.

8. Procédé selon la Revendication 7 caractérisé en ce que l'on utilise un adduit résultant de la réaction de 3 à 9 moles de morpholine sur 1,5 à 4,5 moles d'orthoformiate de méthyle ou d'éthyle, par mole de méthyl-3 nitro-4 pyridine-1-oxyde.

9. Procédé selon la Revendication 7 caractérisé en ce que l'on utilise un adduit résultant de la réaction de 6 moles de morpholine sur 3 moles d'orthoformiate de méthyle ou d'éthyle, par mole de méthyl-3 nitro-4 pyridine-1-oxyde.

10. Enamines de formule générale :

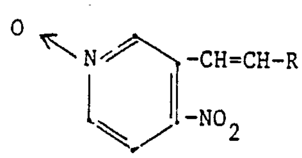

dans laquelle R représente diméthylamino, diéthylamino, dipropylamino, dibutylamino, morpholino, pipéridino ou pyrrolidino en tant que produits industriels pour la préparation de l'aza-5 indole selon l'une des Revendications 1 à 9.

**Claims**

1. Process for preparing 1H-pyrrolo-[3,2-c]-pyridine or 5-aza-indole of formula :

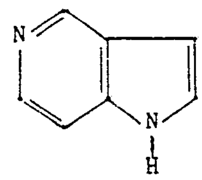

(I)

whereby 3-methyl-4-nitro-pyridine-1-oxyde of formula :

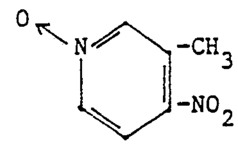

(II)

is condensed, in a solvent or in the absence of a solvent, with a compound of general formula :

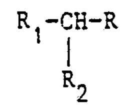

(III)

wherein R represents a dimethylamino, diethylamino, dipropylamino, dibutylamino, morpholino, piperidino or pyrrolidino group and $R_1$ and $R_2$, which are the same or different, each represent a methoxy, ethoxy or propoxy group or a group R as defined above, to provide an enamine of general formula :

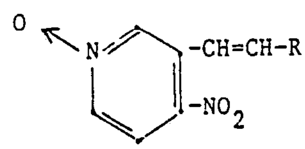

(IV)

in which R has the same meaning as above which is then submitted in a solvent to reduction cyclisation with hydrogen in the presence of a catalyst to obtain the required 5-aza-indole.

2. Process according to Claim 1, whereby the condensation is carried out at a temperature between 0 and 200°C and the reduction cyclisation at a temperature between 0 and 120°C.

3. Process according to Claim 2 whereby the condensation is carried out at a temperature between 100 and 150°C and the reduction cyclisation at a temperature of about 60°C.

4. Process according to Claim 1, whereby the condensation is carried out in N,N-dimethylformamide and the reduction cyclisation in ethanol as solvents.

5. Process according to Claim 1, whereby the catalyst is Raney's nickel or palladium charcoal.

6. Process according to any of Claims 1 to 5, whereby the compound of formula III is N,N-dimethylformamide dimethylacetal, N,N-dimethylformamide diethylacetal or trismorpholinomethane.

7. Process according to Claim 1 whereby, as a source of compound of formula III there is used an adduct obtained by reacting morpholine with methyl or ethyl orthoformiate, this adduct being condensed, in the medium where it is formed, with 3-methyl-4-nitro-pyridine-1-oxyde, to provide the enamine of formula IV.

8. Process according to Claim 7 whereby the adduct used is obtained by reacting 3 to 9 mols of morpholine with 1.5 to 4.5 moles of methyl or ethyl orthoformiate per mol of 3-methyl-4-nitro-pyridine-1-oxide.

9. Process according to Claim 7, whereby the adduct used is obtained by reacting 6 mols of morpholine with 3 mols of methyl or ethyl orthoformiate per mol of 3-methyl-4-nitro-pyridine-1-oxide.

10. Enamines of general formula :

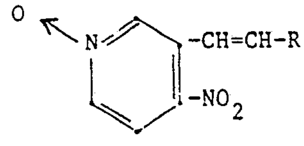

in which R represents a dimethylamino, diethylamino, dipropylamino, dibutylamino, morpholino, piperidino or pyrrolidino group as industrial products for preparing 5-aza-indole in accordance with any of Claims 1 to 9.

**Patentansprüche**

1. Verfahren zur Herstellung von 1H-Pyrrolo-[3,2-c]-pyridin oder 5-Aza-indol der Formel :

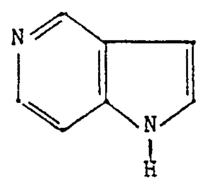

(I)

dadurch gekennzeichnet, daß man 3-Methyl-4-nitro-pyrindin-1-oxid der Formel :

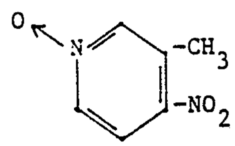

(II)

in einem Lösungsmittel oder in Abwesenheit eines Lösungsmittels mit einer Verbindung der folgenden allgemeinen Formel kondensiert :

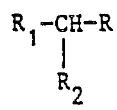

(III)

in der R für eine Dimethylamino-, Diethylamino-, Dipropylamino-, Dibutylamino-, Morpholino-, Piperidino- oder Pyrrolidinogruppe steht und $R_1$ und $R_2$, welche gleich oder unterschiedlich sind, jeweils eine Methoxy-, Ethoxy-, oder Propoxygruppe, oder eine Gruppe R wie oben definiert, darstellen, um ein Enamin der folgenden allgemeinen Formel zu erhalten :

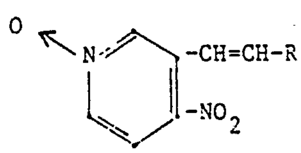

(IV)

in der R dieselbe Bedeutung wie oben hat, worauf man in einem Lösungsmittel in Gegenwart eines Katalysators eine reduktive Cyclisierung mit Wasserstoff durchführt, um das gewünschte 5-Aza-indol zu erhalten.

2. Verfahren nach Anspruch 1, dadurch gekennzeichnet, daß die Kondensation bei einer Temperatur von 0 bis 200°C und die reduktive Cyclisierung bei einer Temperatur von 0 bis 120°C durchgeführt wird.

3. Verfahren nach Anspruch 2, dadurch gekennzeichnet, daß die Kondensation bei einer Temperatur von 100 bis 150°C und die reduktive Cyclisierung bei einer Temperatur von ca. 60°C durchgeführt wird.

4. Verfahren nach Anspruch 1, dadurch gekennzeichnet, daß die Kondensation in N,N-Dimethylformamid und die reduktive Cyclisierung in Ethanol als Lösungsmitteln durchgeführt wird.

5. Verfahren nach Anspruch 1, dadurch gekennzeichnet, daß der Katalysator Raney-Nickel oder Palladium-auf-Aktivkohle ist.

6. Verfahren nach irgendeinem der Ansprüche 1 bis 5, dadurch gekennzeichnet, daß die Verbindung der Formel III N,N-Dimethylformamid-dimethylacetal, N,N-Dimethylformamid-diethylacetal oder Trismorpholinomethan ist.

7. Verfahren nach Anspruch 1, dadurch gekennzeichnet, daß als Quelle für die Verbindung der Formel III ein Addukt verwendet wird, welches durch Reaktion von Morpholin mit Methyloder Ethylorthoformiat erhalten wird, welches man in dem Medium, in dem es gebildet wird, mit 3-Methyl-4-nitro-pyridin-1-oxid kondensiert, um das Enamin der Formel IV zu bilden.

8. Verfahren nach Anspruch 7, dadurch gekennzeichnet, daß man ein Addukt verwendet, das durch Reaktion von 3 bis 9 Mol Morpholin mit 1,5 bis 4,5 Mol Methyl- oder Ethylorthoformiat pro Mol 3-Methyl-4-nitro- pyridin-1-oxid erhalten wird.

9. Verfahren nach Anspruch 7, dadurch gekennzeichnet, daß man ein Addukt verwendet, das durch Reaktion von 6 Mol Morpholin mit 3 Mol Methyl- oder Ethylorthoformiat pro Mol 3-Methyl-4-nitro-pyridin-1-oxid erhalten wird.

10. Enamine der allgemeinen Formel :

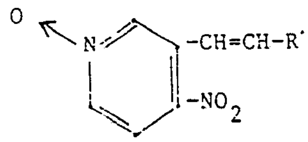

in denen R für eine Dimethylamino-, Diethylamino-, Dipropylamino-, Dibutylamino-, Morpholino-, Piperidino- oder Pyrrolidinogruppe steht, als industrielle Produkte zur Herstellung von 5-Aza-indol gemäß einem der Ansprüche 1 bis 9.